# EUROPÄISCHE PATENTSCHRIFT

(11) **EP 2 545 857 B1**
(45) Veröffentlichungstag und Bekanntmachung des Hinweises auf die Patenterteilung: **31.12.2014**
(21) Anmeldenummer: 12005050.5
(22) Anmeldetag: 07.07.2012
(51) Int. Cl.: A61B 17/00, A61B 17/32, A61B 17/295

(54) **Medizinisches Schneidinstrument zum Schneiden von Muskeln und Sehnen**
Medical cutting instrument for cutting muscles and tendons
Instrument de coupe médical destiné à découper des muscles et des tendons

(30) Priorität: 13.07.2011 DE 102011107178
(43) Veröffentlichungstag der Anmeldung: 16.01.2013
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Hofmeister, Frank

(56) Entgegenhaltungen:
- WO-A1-03/011152
- US-A- 2 706 987
- US-A- 3 763 860
- US-A- 4 955 897
- US-A- 5 071 424
- US-A- 5 196 023
- US-A- 5 254 129
- US-A- 5 304 203
- US-A- 6 024 744
- US-A- 6 077 280
- US-A1- 2006 258 954

## Beschreibung

Die Erfindung betrifft ein medizinisches Schneidinstrument zum Schneiden von Muskeln und Sehnen, mit einem Schaft, an dessen distalem Ende ein aus zwei Maulteilen bestehendes Werkzeug angeordnet ist, wobei wenigstens ein Maulteil als gegenüber dem anderen Maulteil verstellbares Maulteil ausgebildet ist, und an dessen proximalem Ende eine Handhabe angeordnet ist, wobei das verstellbare Maulteil und die Handhabe über ein im Schaft gelagertes Betätigungselement derart in Wirkverbindung miteinander stehen, dass das verstellbare Maulteil durch Betätigen der Handhabe zwischen einer geschlossenen Position und einer geöffneten Position des Werkzeuges verstellbar ist und wobei das Werkzeug zum gleichzeitigen Durchtrennen und Halten eines Endes des durchtrennten Muskel-/Sehnengewebes ausgebildet ist.

Gattungsgemäße medizinische Schneidinstrumente werden beispielsweise bei der Bizepstenotomie- oder Bizepstenodese-Operation verwendet, um die Bizepssehne zu durchtrennen, wenn diese aufgrund von Abnutzung Schmerzen im Gelenk hervorruft.

Aus der DE 100 03 020 A1 ist ein bipolares medizinisches Fassinstrument bekannt, dessen Werkzeug zum gleichzeitigen Durchtrennen und Halten eines Endes des durchtrennten Muskel-/Sehnengewebes ausgebildet ist. Bei diesem bekannten, zum Halten und Schneiden von Gefäßen ausgebildeten medizinischen Instrument ist die Schneidvorrichtung in der Mitte der Klemmflächen der Maulteile angeordnet. Die Anordnung der Schneidvorrichtung sowie der Klemmflächen der Maulteile ermöglicht jedoch kein kontrolliertes Abtrennen von Muskel- und Sehnengewebe.

Ein weiteres medizinisches Schneidinstrument zum Schneiden von Muskeln und Sehnen ist beispielsweise aus dem US-Patent US 253,359 A bekannt. Mit diesem bekannten Instrument ist es möglich, eine Inzision zu schaffen, die eine Sehne freilegt, diese Sehne zu bergen sowie ein Stück aus dem Sehnengewebe herauszutrennen.

US 6,024,744 offenbart ein chirurgisches Schneide- und Greifeinstrument mit den Merkmalen der Präambel von Anspruch 1.

Im Gegensatz zur offenen Chirurgie, bei der die Sehne blind abgeschnitten wird, da man den Situs nicht eröffnen will, ist die Sehne bei der Arthroskopie zwar sichtbar, muss aber vor dem Durchtrennen zunächst durch Anschlingen und arthroskopisches Nähen gesichert werden, um zu verhindern, dass sich die Sehne nach dem Durchtrennen sofort zum Ansatz zurückzieht und dann mittels offener Chirurgie wieder geborgen werden muss.

Davon ausgehend liegt der Erfindung die **Aufgabe** zugrunde, ein medizinisches Schneidinstrument der eingangs genannten Art zu schaffen, das bei einfacher Handhabung ein kontrolliertes Durchtrennen des Muskel-/Sehnengewebes ermöglicht.

Die **Lösung** dieser Aufgabenstellung ist erfindungsgemäß gekennzeichnet durch die Merkmale des Anspruchs 1 gegeben. Hierbei ist zumindest im anderen Maulteil eine Führung zur führenden Aufnahme des zu durchtrennenden Muskel-/Sehnengewebes ausgebildet.

Vorteilhafte Weiterbildungen der Erfindung sind Gegenstand der Unteransprüche.

Durch die Ausgestaltung des Werkzeugs als kombiniertes Schneid- und Haltewerkzeug ist es möglich, ein Ende des im ersten Arbeitsschritt mit demselben Instrument durchtrennten Muskel- oder Sehnengewebes klemmend festzuhalten und so am Zurückziehen zu hindern.

Um sicherzustellen, dass das zu durchtrennende Muskel-/Sehnengewebe richtig zur Schneidkante und zu den Klemmflächen ausgerichtet ist, sind zumindest im anderen Maulteil, vorzugsweise in beiden Maulteilen, Führungen zur führenden Aufnahme des zu durchtrennenden Muskel-/Sehnengewebes ausgebildet.

Gemäß der Erfindung wird vorgeschlagen, dass zum Halten des durchtrennten Muskel- oder Sehnengewebes am beiden Maulteilen miteinander korrespondierende Klemmflächen angeordnet sind, wobei die Klemmflächen vorzugsweise als quer zur Instrumentenlängsachse ausgerichtete ineinander greifende Verzahnungen ausgebildet sind.

Mit einer alternativen Ausführungsform der Erfindung wird vorgeschlagen, dass die Klemmflächen als durch Quer- und Längsvertiefungen voneinander getrennte Einzelzähne ausgebildet sind. Die Ausbildung als Einzelzähne gewährt einen sicheren Halt und eine gute seitliche Verrutschsicherheit für das durchtrennte Muskel- oder Sehnengewebe.

Weiterhin wird mit der Erfindung vorgeschlagen, dass die auf den Klemmflächen ausgebildete quer ausgerichtete Verzahnung bzw. die Einzelzähne zumindest anteilig nach distal geneigt ausgebildet sind, um sicherzustellen, dass ein besseres Verkeilen und Festhalten des durchtrennten Muskel- oder Sehnengewebes erreicht wird, da durch die Neigung nach distal das Gewebe, das sich nach proximal zurückziehen will, fester gegen die Verzahnung bzw. die Einzelzähne gepresst wird. Diese Wirkung wird zunehmender Anzahl der geneigten Verzahnungen bzw. Einzelzähne der Klemmflächen verstärkt.

Weiterhin wird mit der Erfindung vorgeschlagen, die Klemmflächen als am starren Maulteil angeordneter solitärer Einzelzahn und als am verschwenkbaren Maulteil entlang der Ausnehmung verlaufende Zahnreihe ausgebildet ist.

Zum Ausbilden der Schneidfunktion ist zumindest am verstellbaren Maulteil eine Schneidkante ausgebildet, die nach unten zum anderen Maulteil hin ausgerichtet am distalen Ende des verstellbaren Maulteils angeordnet ist.

Mit einer alternativen Ausführungsform der Erfindung wird vorgeschlagen, dass die Schneidkante nach unten zum anderen Maulteil hin ausgerichtet U-förmig ausgebildet so am verstellbaren Maulteil angeordnet ist, dass sich die Schneidkante vom distalen Ende des verstellbaren Maulteils zu beiden Seitenbereichen des verstellbaren Maulteils erstreckt. Diese Ausgestaltung der Schneidkante hat den Vorteil, dass gerade bei schwierigen Schneidsituationen, beispielsweise durch ungewöhnlich dicke Sehnen oder durch die Sehnenscheide, ein sicheres Schneidergebnis erzielbar ist.

Um ein präzises und vollständiges Durchtrennen des Muskel- oder Sehnengewebes beim Schließen des verstellbaren Maulteils zu gewährleisten, wird mit der Erfindung vorgeschlagen, dass im Bereich der am verstellbaren Maulteil angeordneten Schneidkante im anderen Maulteil eine Aussparung zur Aufnahme der Schneidkante ausgebildet ist, die beispielsweise als Vertiefung oder durchgehende Öffnung im starren Maulteil ausgebildet ist.

Weiterhin wird mit der Erfindung vorgeschlagen, dass die Klemmfläche am verstellbaren Maulteil proximal hinter der Schneidkante angeordnet ist und/oder die Klemmfläche am anderen Maulteil proximal hinter der Aussparung zur Aufnahme der Schneidkante angeordnet ist, um ein Ende des durchtrennten Gewebes direkt nach dem Durchtrennen klemmend fixieren zu können.

Schließlich wird mit der Erfindung vorgeschlagen, dass das distale Ende des anderen Maulteils, das das verstellbare Maulteil in axialer Richtung überragt, abgerundet ausgebildet ist, um durch die dilatatorförmige Ausgestaltung der distalen Instrumentenspitze das Eindringen des medizinischen Schneidinstruments durch Weichteile zu erleichtern.

Zur Einstellung und Verstellung der Schneidtiefe wird mit der Erfindung vorgeschlagen, dass im Bereich der Handhabe ein Hebelmechanismus vorgesehen ist, der einen variablen Anschlag für das Schließen der beiden Maulteile gegeneinander ausbildet und so die Verstellung der Schneidtiefe bewirkt.

Weiterhin wird mit der Erfindung vorgeschlagen, dass an der Handhabe eine Skalierung angeordnet ist, um das Muskel-/Sehnengewebe gezielt in einer bestimmten Tiefe durchtrennen zu können.

Weitere Merkmale und Vorteile der Erfindung ergeben sich anhand der zugehörigen Zeichnungen, in denen vier Ausführungsbeispiele eines erfindungsgemäßen medizinischen Schneidinstruments zum Schneiden von Muskeln und Sehnen nur beispielhaft dargestellt sind, ohne die Erfindung auf diese Ausführungsbeispiele zu beschränken. In den Zeichnungen zeigt:
- Fig. 1: eine schematische Seitenansicht einer ersten Ausführungsform eines erfindungsgemäßen medizinischen Schneidinstruments zum Schneiden von Muskeln und Sehnen;
- Fig. 2: eine vergrößerte perspektivische Draufsicht auf das Detail II gemäß Fig. 2;
- Fig. 3: eine Ansicht von unten der Darstellung gemäß Fig. 2;
- Fig. 4: eine Ansicht gemäß Fig. 2, jedoch die geschlossene Position einer zweiten erfindungsgemäßen Ausführungsform darstellend;
- Fig. 5: eine perspektivische Ansicht einer Klemmfläche gemäß einer dritten erfindungsgemäßen Ausführungsform;
- Fig. 6: eine perspektivische Vorderansicht eines Werkzeugs gemäß einer vierten erfindungsgemäßen Ausführungsform und
- Fig. 7: eine perspektivische Rückansicht des Werkzeugs gemäß Fig. 6.

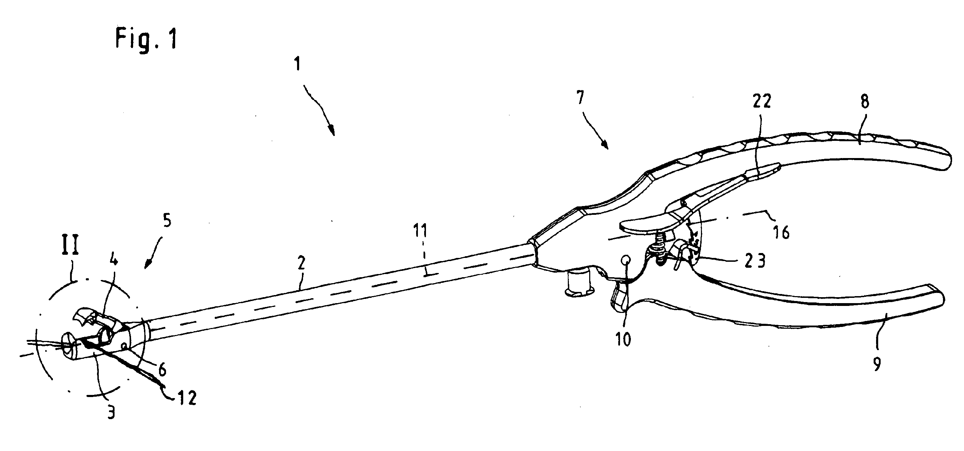

Die Abbildung Fig. 1 zeigt eine perspektivische Ansicht eines medizinischen Schneidinstruments 1 zum Schneiden von Muskeln und Sehnen.

Dieses beispielsweise als Bizepssehnenstanze ausgebildete und sowohl für die offene Chirurgie als auch für die arthroskopische Chirurgie verwendbare Schneidinstrument 1 weist einen Schaft 2 auf, an dessen distalem Ende ein aus zwei Maulteilen 3 und 4 bestehendes Werkzeug 5 angeordnet ist, wobei ein Maulteil 3 starr ausgebildet ist und das andere Maulteil 4 als um eine Schwenkachse 6 gegenüber dem starren Maulteil 3 verschwenkbares Maulteil 4 ausgebildet ist. Am proximalem Ende des Schaftes 2 ist eine Handhabe 7 angeordnet, die bei der dargestellten Ausführungsform aus zwei Griffteilen 8 und 9 besteht, wobei ein Griffteil 8 starr ausgebildet ist und das andere Griffteil 9 als um eine Schwenkachse 10 gegenüber dem starren Griffteil 8 verschwenkbares Griffteil 9 ausgebildet ist.

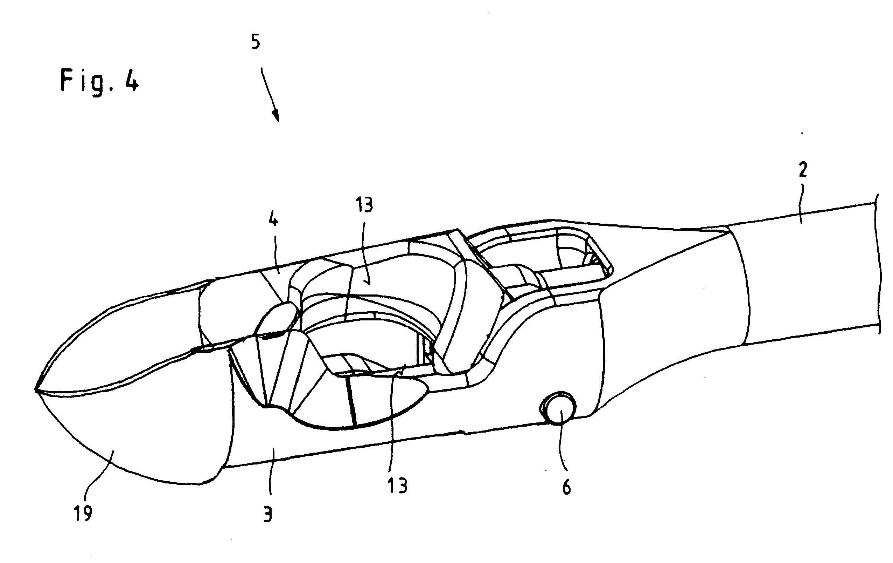

Das verschwenkbare Maulteil 4 des Werkzeugs 5 und das verschwenkbare Griffteil 9 der Handhabe 7 stehen über ein im Schaft 2 gelagertes Betätigungselement 11 derart in Wirkverbindung miteinander, dass das verschwenkbare Maulteil 4 durch Betätigen des verschwenkbaren Griffteils 9 zwischen einer am starren Maulteil 3 anliegenden geschlossenen Position (Fig. 4, 6 und 7) und einer gegenüber dem starren Maulteil 3 verschwenkten offenen Position (Fig. 1 bis 3) verstellbar ist. Durch das starre Maulteil 3 kann eine besonders vorteilhafte Führung und damit Positionierung der Sehne beim Schnitt erreicht werden, was bei der subkutanen Anwendung des erfindungsgemäßen Instrumentes zu einem wesentlich besseren Schneidergebnis führt.

Alternativ zu der dargestellten Ausführungsform des Schneidinstruments 1 mit einem starren Maulteil 3 und einem verschwenkbaren Maulteil 4 ist es selbstverständlich auch möglich, beide Maulteile verschwenkbar auszugestalten. Gerade für freischwebende, freipräparierbare Sehnen erweist sich eine Variante mit zwei verschwenkbaren Maulteilen als besonders sinnvoll.

Weiterhin ist das dargestellte Verschwenken des Maulteils 4 gegenüber dem anderen Maulteil 3 nur eine Ausführungsform zum gegenseitigen Verstellen der Maulteile 3 und 4 zueinander. Neben dem Verschwenken um die Schwenkachse 6 ist es beispielsweise auch möglich, die Maulteile 3 und 4 axial gegeneinander zu verschieben.

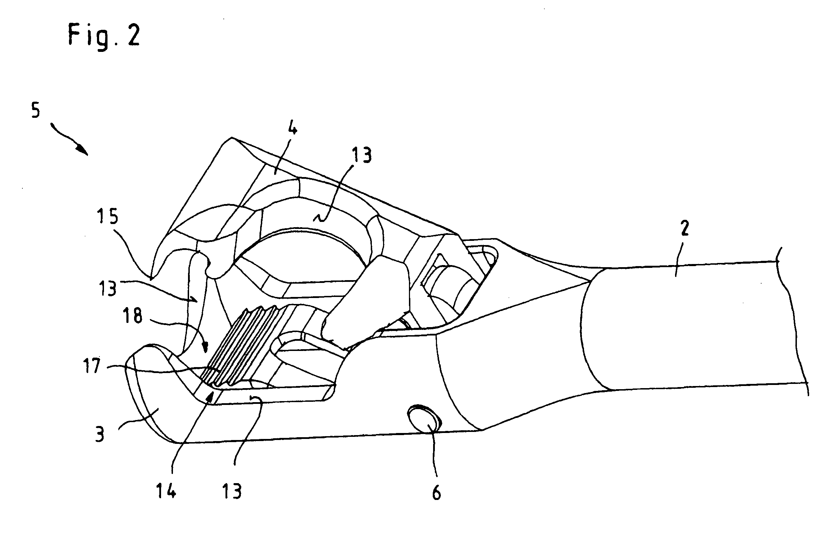

Der Aufbau des aus den Mauteilen 3 und 4 bestehenden distalseitigen Werkzeugs 5 ist insbesondere den vergrößerten Detailansichten der Abbildungen Fig. 2 bis 4 sowie 6 und 7 zu entnehmen.

Zur führenden Aufnahme des zu schneidenden Muskel-/Sehnengewebes 12 (Fig. 1) sind bei den dargestellten Ausführungsformen in beiden Maulteilen 3 und 4 als Ausnehmungen 13 ausgebildete Führungen 13 angeordnet.

Die Besonderheit der dargestellten Werkzeuge 5 ist, dass diese Werkzeuge 5 zum gleichzeitigen Durchtrennen und Halten eines Endes des durchtrennten Muskel-/Sehnengewebes 12 ausgebildet ist. Auf dieses Weise kann verhindert werden, dass sich das durchtrennte Muskel-/Sehnengewebe 12 nach dem Durchtrennen zum Ansatz hin zurückzieht, wenn das Muskel-/Sehnengewebe 12 nicht zuvor, beispielsweise durch arthroskopisches Nähen, gesichert wurde.

Zur Ausbildung dieser Klemm-Schneid-Doppelfunktion sind einerseits am starren Maulteil 3 und am verschwenkbaren Maulteil 4 miteinander korrespondierende Klemmflächen 14 angeordnet und ist andererseits zumindest am verschwenkbaren Maulteil 4 eine Schneidkante 15 ausgebildet. Die Klemmflächen 14 sind bei diesen Ausführungsformen als quer zur Instrumentenlängsachse 16 ausgerichtete Verzahnungen 17 ausgebildet sind. Das Ineinandergreifen der Verzahnungen 17 gewährleistet einen sicheren und rutschfesten Halt des zwischen den Klemmflächen 14 angeordneten Muskel-/Sehnengewebe 12.

Die Schneidkante 15 ist nach unten zum starren Maulteil 3 hin ausgerichtet so am distalen Ende des verschwenkbaren Maulteils 4 angeordnet, dass das in den Führungen 13 der Maulteile 3 und 4 angeordnete Muskel-/Sehnengewebe 12 durchtrennt wird, sobald das verschwenkbare Maulteil 4 des Werkzeugs 5 vollständig geschlossen wird. Um weiterhin sicher zu stellen, dass nach dem Durchtrennen des Muskel-/Sehnengewebes 12 mittels der Schneidkante 15 das im proximalen Teil des Werkzeugs 5 befindliche Ende des durchtrennten Muskel-/Sehnengewebes 12 sicher von den miteinander korrespondierende Klemmflächen 14 der Maulteile 3 und 4 gehalten wird, ist im Bereich der am verschwenkbaren Maulteil 4 angeordneten Schneidkante 15 im starren Maulteil 3 eine als Öffnung 18 ausgebildete Aussparung 18 zur Aufnahme der Schneidkante 15 angeordnet.

Diese Aussparung 18 im starren Maulteil 3 des Werkzeugs 5 stellt sicher, dass das verschwenkbare Maulteil 4 des Werkzeugs 5 vollständig geschlossen werden kann und nicht durch das Anlaufen der Scheidkante 15 gegen das starre Maulteil 3 gestoppt wird. Alternativ zur Ausbildung der Aussparung 18 als durchgehende Öffnung 18 ist es selbstverständlich auch möglich, die Aussparung 18 als nicht offene Vertiefung im starren Maulteil 3 auszubilden.

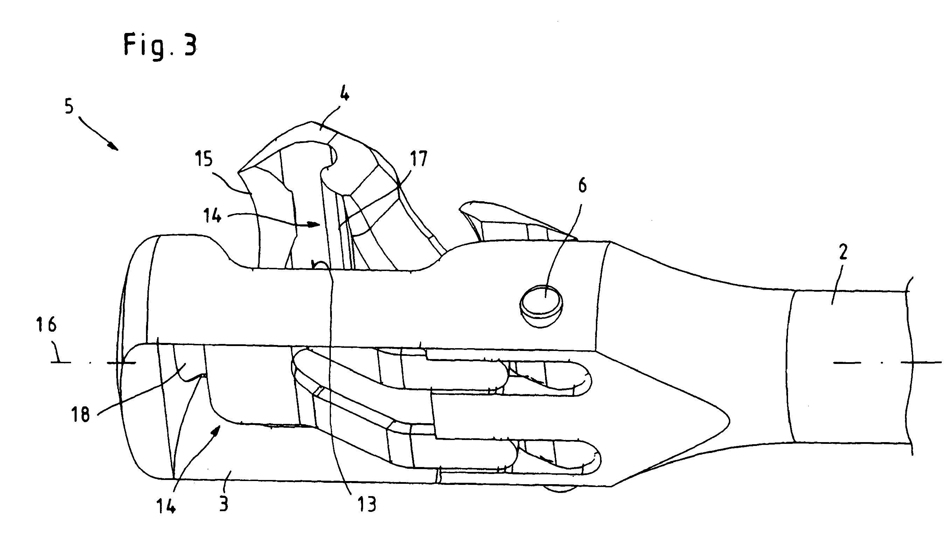

Die Platzierung der Klemmflächen 14 an den Maulteilen 3 und 4 ergibt sich aus einer Zusammenschau der Abbildungen Fig. 2 und 3, wonach die Klemmfläche 14 am verschwenkbaren Maulteil 4 proximal hinter der Schneidkante 15 angeordnet ist und die Klemmfläche 14 am starren Maulteil 3 proximal hinter der Aussparung 18 angeordnet ist.

Die Abbildung Fig. 4 zeigt eine zweite, alternative Ausführungsform zur Ausbildung des Werkzeugs 5.

Bei dieser alternativen Ausführungsform ist zusätzlich das distale Ende 19 des starren Maulteils 3, das das verschwenkbare Maulteil 4 in axialer Richtung überragt, abgerundet ausgebildet ist, um durch die dilatatorförmige Ausgestaltung der distalen Instrumentenspitze das Eindringen des medizinischen Schneidinstruments 1 durch Weichteile zu erleichtern.

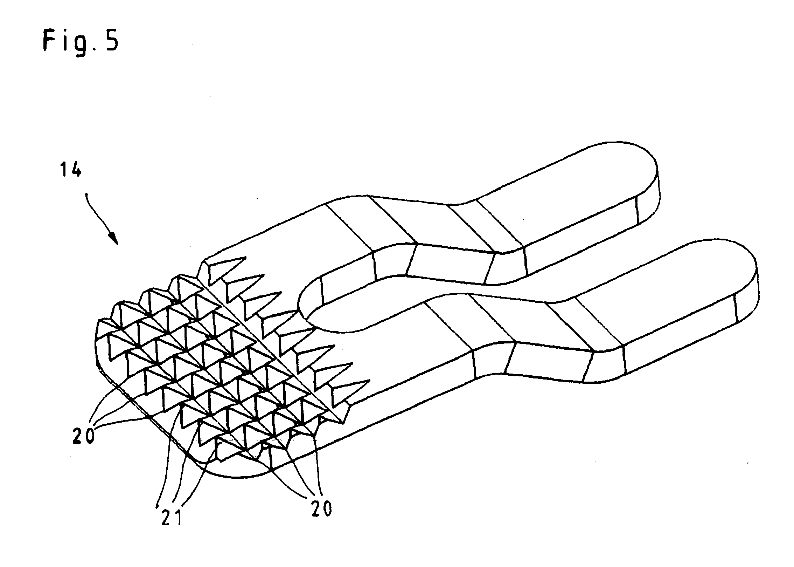

Die Abbildung Fig. 5 zeigt eine alternative Ausführungsform zur Ausbildung der Klemmflächen 14. Bei dieser Ausgestaltungsform sind die Klemmflächen 14 als durch Quer- und Längsvertiefungen 20 voneinander getrennte Einzelzähne 21 ausgebildet. Die Ausbildung als Einzelzähne 21 gewährt einen sicheren Halt und eine gute seitliche Verrutschsicherheit für das durchtrennte Muskel- oder Sehnengewebe 12.

Die auf den Klemmflächen 14 ausgebildete quer ausgerichtete Verzahnung 17 bzw. die Einzelzähne 21 sind vorteilhafterweise nach distal geneigt ausgebildet, um sicherzustellen, dass ein besseres Verkeilen und Festhalten des durchtrennten Muskel- oder Sehnengewebes 12 erreicht wird. Durch die Neigung nach distal wird das Muskel- oder Sehnengewebe 12, das sich nach proximal zurückziehen will, fester gegen die Verzahnung 17 bzw. die Einzelzähne 21 gepresst.

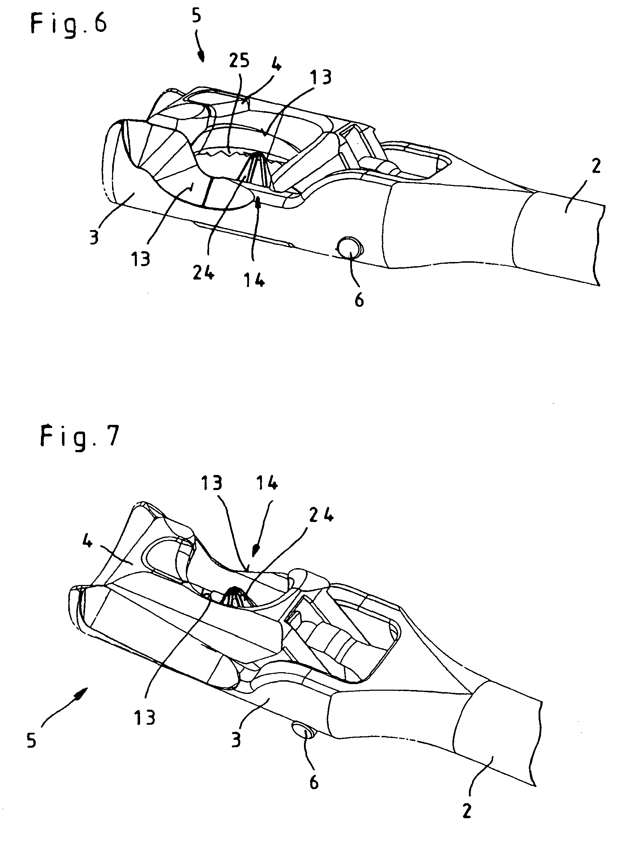

Die Abbildungen Fig. 6 und 7 zeigen schließlich eine alternative Ausführungsform zur Ausbildung der Klemmflächen 14. Bei dieser Ausgestaltungsform ist die Klemmfläche 14 am starren Maulteil 3 als solitärer Einzelzahn 24 ausgebildet, während die Klemmfläche 14 am verschwenkbaren Maulteil 4 als entlang der bogenförmigen Ausnehmung 13 verlaufende Zahnreihe 25 ausgebildet ist.

Der vorzugsweise im Wesentlichen mittig am starren Maulteil 3 angeordnete solitäre Einzelzahn 24 presst das zu haltende Muskel-/Sehnengewebe 12 in der geschlossenen Position der Maulteile 3 und 4 gegen die Zahnreihe 25 des verschwenkbaren Maulteils 4 und gewährleistet so den sicheren Halt des durchtrennten Muskel-/Sehnengewebes 12.

Wie weiterhin aus Fig. 1 ersichtlich, ist zur Einstellung und Verstellung der schneidtiefe an der Handhabe 7 ein Hebelmechanismus 22 vorgesehen, der einen variablen Anschlag für das Schließen der beiden Maulteile 3 und 4 gegeneinander ausbildet und so die Verstellung der Schneidtiefe bewirkt.

Weiterhin ist an der Handhabe 7 eine von außen ablesbare Skalierung 23 vorgesehen, damit das Muskel-/Sehnengewebe durch die Betätigung des Hebelmechanismus 22 gezielt in einer bestimmten Tiefe durchtrennt werden kann.

Durch die Ausgestaltung des voranstehen beschriebenen medizinischen Schneidwerkzeugs 1 als kombiniertes Schneid- und Haltewerkzeug ist es möglich, ein Ende des im ersten Arbeitsschnitt mit demselben Schneidinstrument 1 durchtrennten Muskel- oder Sehnengewebes 12 klemmend festzuhalten und so am Zurückziehen zu hindern und zur weiteren operativen Verwendung bereit zu halten.

**Bezuguszeichenliste**

| | | | |
|---|---|---|---|
| 1 | medizinisches Schneidinstrument | 18 | Aussparung / Öffnung |
| 2 | Schaft | 19 | distales Ende |
| 3 | starres / anderes Maulteil | 20 | Quer-/Längsvertiefung |
| 4 | verschwenkbares / verstellbares Maulteil | 21 | Einzelzahn |
| 5 | Werkzeug | 22 | Hebelmechanismus |
| 6 | Schwenkachse | 23 | Skalierung |
| 7 | Handhabe | 24 | Einzelzahn |
| 8 | starres Griffteil | 25 | Zahnreihe |
| 9 | verschwenkbares Griffteil | | |
| 10 | Schwenkachse | | |
| 11 | Betätigungselement | | |
| 12 | Muskel-/Sehnengewebe | | |
| 13 | Ausnehmung / Führung | | |
| 14 | Klemmfläche | | |
| 15 | Schneidkante | | |
| 16 | Instrumentenlängsachse | | |
| 17 | Verzahnung | | |

## Patentansprüche

1. Medizinisches Schneidinstrument zum Schneiden von Muskeln und Sehnen, mit einem Schaft (2), an dessen distalem Ende ein aus zwei Maulteilen (3, 4) bestehendes Werkzeug (5) angeordnet ist, wobei wenigstens ein erstes Maulteil (4) als gegenüber dem anderen zweiten Maulteil (3) verstellbares erstes Maulteil (4) ausgebildet ist, und an dessen proximalem Ende eine Handhabe (7) angeordnet ist, wobei das verstellbare erste Maulteil (4) und die Handhabe (7) über ein im Schaft (2) gelagertes Betätigungselement (11) derart in Wirkverbindung miteinander stehen, dass das verstellbare erste Maulteil (4) durch Betätigen der Handhabe (7) zwischen einer geschlossenen Position und einer geöffneten Position des Werkzeuges (5) verstellbar ist und wobei das Werkzeug (5) zum gleichzeitigen Durchtrennen und Halten eines Endes des durchtrennten Muskel-/Sehnengewebes (12) ausgebildet ist,
wobei zumindest am verstellbaren ersten Maulteil (4) eine zum zweitenMaulteil (3) hin ausgerichtete und am distalen Ende des verstellbaren ersten Maulteils (4) angeordnete Schneidkante (15) ausgebildet ist sowie an beiden Maulteilen (3, 4) miteinander korrespondierende Klemmflächen (14) angeordnet sind **dadurch gekennzeichnet, dass** zumindest im zweiten Maulteil (3) eine Führung (13) zur führenden Aufnahme des zu durchtrennenden Muskel-/Sehnengewebes (12) ausgebildet ist, die dazu geeignet ist, das zu dauchtrennende Muskel-/Sehnengewebe (12) zur Schneidkonte (15) und zu den Klemmflächen (14) auszwichten.

2. Medizinisches Schneidinstrument nach Anspruch 1, **dadurch gekennzeichnet, dass** die Führung (13) in beiden Maulteilen (3, 4) ausgebildet ist.

3. Medizinisches Schneidinstrument nach Anspruch 1 oder 2, **dadurch gekennzeichnet, dass** die Klemmflächen (14) als quer zur Instrumentenlängsachse (16) ausgerichtete Verzahnungen (17) ausgebildet sind.

4. Medizinisches Schneidinstrument nach Anspruch 1 oder 2, **dadurch gekennzeichnet, dass** die Klemmflächen (14) als durch Quer- und Längsvertiefungen (20) voneinander getrennte Einzelzähne (21) ausgebildet sind.

5. Medizinisches Schneidinstrument nach Anspruch 3 oder 4, **dadurch gekennzeichnet, dass** die quer ausgerichtete Verzahnung (17) und/oder die Einzelzähne (21) zumindest anteilig nach distal geneigt ausgebildet sind.

6. Medizinisches Schneidinstrument nach Anspruch 1, **dadurch gekennzeichnet, dass** die Klemmflächen (14) als am starren Maulteil (3) angeordneter solitärer Einzelzahn (24) und als am verschwenkbaren Maulteil (4) entlang der Ausnehmung (13) verlaufende Zahnreihe (25) ausgebildet ist.

7. Medizinisches Schneidinstrument nach einem der Ansprüche 1 bis 6, **dadurch gekennzeichnet, dass** die Schneidkante (15) zum anderen Maulteil (3) hin ausgerichtet U-förmig ausgebildet so am verstellbaren Maulteil (4) angeordnet ist, dass sich die Schneidkante (15) vom distalen Ende des verstellbaren Maulteils (4) zu beiden Seitenbereichen des verstellbaren Maulteils (4) erstreckt.

8. Medizinisches Schneidinstrument nach Anspruch einem der Ansprüche 1 bis 6, **dadurch gekennzeichnet, dass** im Bereich der am verstellbaren Maulteil (4) angeordneten Schneidkante (15) im anderen Maulteil (3) eine Aussparung (18) zur Aufnahme der Schneidkante (15) ausgebildet ist.

9. Medizinisches Schneidinstrument nach Anspruch 8, **dadurch gekennzeichnet, dass** die Aussparung (18) als Vertiefung oder durchgehende Öffnung (18) ausgebildet ist.

10. Medizinisches Schneidinstrument nach Anspruch 8 oder 9, **dadurch gekennzeichnet, dass** die Klemmfläche (14) am verstellbaren Maulteil (4) proximal hinter der Schneidkante (15) angeordnet ist und/oder die Klemmfläche (14) am anderen Maulteil (3) proximal hinter der Aussparung (18) angeordnet ist.

11. Medizinisches Schneidinstrument nach einem der Ansprüche 1 bis 10, **dadurch gekennzeichnet, dass** das distale Ende (19) des anderen Maulteils (3), das das verstellbare Maulteil (4) in axialer Richtung überragt, abgerundet ausgebildet ist.

12. Medizinisches Schneidinstrument nach einem der Ansprüche 1 bis 11, **dadurch gekennzeichnet, dass** im Bereich der Handhabe (7) ein Hebelmechanismus (22) angeordnet ist, der einen variablen Anschlag für das Schließen der Maulteile (3 und 4) gegeneinander bildet und so eine Verstellung der Schneidtiefe bewirkt.

13. Medizinisches Schneidinstrument nach Anspruch 12, **dadurch gekennzeichnet, dass** an der Handhabe (7) eine Skalierung (23) zur Bestimmung der Schnitttiefe angeordnet ist.

## Claims

1. Medical cutting instrument for cutting muscles and tendons, with a shaft (2) on the distal end of which a tool (5) consisting of two jaw parts (3, 4) is disposed, wherein at least a first jaw part (4) is formed as a first jaw part (4) movable relative to the other, second jaw part (3), and at the proximal end thereof a handle (7) is disposed, wherein the movable first jaw part (4) and the handle (7) are operatively connected with one another over an actuation element (11) mounted in a shaft (2) in such a manner that the first, movable jaw part (4) can be moved between a closed position and an open position of the tool (5) by actuating the handle (7), and wherein the tool (5) is designed for simultaneously cutting and holding an end of the severed muscle/ tendon tissue (12), wherein at least on the movable first jaw part (4) a cutting edge (15) aligned with the second jaw part (3) and located on the distal end of the movable first jaw part (4) is formed and clamping surfaces (14) corresponding to one another are disposed on the two jaw parts (3, 4), **characterized in that** at least in the second jaw part (3) a guideway (13) is formed for accommodating and guiding the muscle/tendon tissue (12) to be cut, which is suitable for aligning the muscle/tendon to be cut with the cutting edge (15) and the clamping surfaces (14).

2. Medical cutting instrument according to claim 1, **characterized in that** the guideway (13) is formed in both jaw parts (3, 4).

3. Medical cutting instrument according to claim 1 or 2, **characterized in that** the clamping surfaces (14) are formed as tooth systems (17) aligned perpendicularly with the longitudinal axis (16) of the instrument.

4. Medical cutting instrument according to claim 1 or 2, **characterized in that** the clamping surfaces (14) are formed as individual teeth (21) separated from one another by transverse and longitudinal indentations (20).

5. Medical cutting instrument according to claim 3 or 4, **characterized in that** the transversely aligned clamping tooth system (17) and/or the individual teeth (21) are formed to be at least partially inclined distally.

6. Medical cutting instrument according to claim 1, **characterized in that** the clamping surfaces (14) are formed as a solitary single tooth (24) disposed on the rigid jaw part (3) and as a row of teeth (25) running on the pivotable part of the jaw (4) along the recess (13).

7. Medical cutting instrument according to one of claims 1 to 6, **characterized in that** the cutting edge (15) is disposed on the movable jaw part (4) in U-shaped alignment relative to the other jaw part (3) extending from the distal end of the movable jaw part (4) toward both side areas of the movable jaw part (4).

8. Medical cutting instrument according to one of claims 1 to 6, **characterized in that** in the area of the cutting edge (15) disposed on the movable jaw part (4), a hollow (18) is formed in the other jaw part (3) to receive the cutting edge (15).

9. Medical cutting instrument according to claim 8, **characterized in that** the hollow (18) is formed as an indentation or a penetrating opening (18).

10. Medical cutting instrument according to claim 8 or 9, **characterized in that** the clamping surface (14) is disposed on the movable jaw part (4) proximally behind the cutting edge (15) and/or the clamping surface (14) is disposed on the other jaw part (3) proximally behind the hollow (18).

11. Medical cutting instrument according to one of claims 1 to 10, **characterized in that** the distal end (19) of the other jaw part (3), which projects beyond the movable jaw part (4) in the axial direction, is made to be rounded.

12. Medical cutting instrument according to one of claims 1 to 11, **characterized in that** in the area of the handle (7) a lever mechanism (22) is disposed, which forms a variable stop for the closing of the jaw parts (3 and 4) against one another and thus makes it possible to adjust the cutting depth.

13. Medical cutting instrument according to claim 12, **characterized in that** a scale (23) is disposed on the handle (7) for determining the cutting depth.

## Revendications

1. Instrument de coupe médical destiné à couper des muscles et des tendons, comprenant un corps d'instrument (2), à l'extrémité distale duquel est agencé un outil (5) constitué de deux mâchoires (3, 4) dont au moins une première mâchoire (4) est réalisée en tant que première mâchoire (4) pouvant être déplacée par rapport à l'autre, deuxième, mâchoire (3), et à l'extrémité proximale duquel est agencée une poignée de manoeuvre (7), instrument
dans lequel la première mâchoire déplaçable (4) et la poignée de manoeuvre (7) sont en interaction mutuelle par l'intermédiaire d'un élément d'actionnement (11) monté dans le corps d'instrument (2), de manière telle que la première mâchoire déplaçable (4) puisse être déplacée, par actionnement de la poignée de manoeuvre (7), entre une position fermée et une position ouverte de l'outil (5), et
dans lequel l'outil (5) est conçu et réalisé pour permettre le sectionnement et le maintien simultané d'une extrémité des tissus de muscles/tendons (12) ayant été sectionnés, et
dans lequel sur au moins la première mâchoire déplaçable (4), est formée une arête de coupe (15) orientée vers la deuxième mâchoire (3) et agencée à l'extrémité distale de la première mâchoire déplaçable (4), et sur les deux mâchoires (3, 4) sont agencées des surfaces de serrage (14) mutuellement correspondantes,
**caractérisé**
**en ce qu'**au moins dans la deuxième mâchoire (3) est formé un guidage (13), qui est destiné à accueillir de manière guidée les tissus de muscles/tendons (12) à sectionner, et est adapté à orienter les tissus de muscles/tendons (12) par rapport à l'arête de coupe (15) et aux surfaces de serrage (14).

2. Instrument de coupe médical selon la revendication 1, **caractérisé en ce que** le guidage (13) est formé dans les deux mâchoires (3, 4).

3. Instrument de coupe médical selon la revendication 1 ou la revendication 2, **caractérisé en ce que** les surfaces de serrage (14) sont réalisées sous forme de dentures (17) orientées transversalement à l'axe longitudinal (16) de l'instrument.

4. Instrument de coupe médical selon la revendication 1 ou la revendication 2, **caractérisé en ce que** les surfaces de serrage (14) sont réalisées sous forme de dents individuelles (21) séparées les unes des autres par des creux transversaux et longitudinaux (20).

5. Instrument de coupe médical selon la revendication 3 ou la revendication 4, **caractérisé en ce que** la denture (17) orientée transversalement et/ou les dents individuelles (21) sont réalisées, au moins en partie, de manière inclinée en direction distale.

6. Instrument de coupe médical selon la revendication 1, **caractérisé en ce que** les surfaces de serrage (14) sont réalisées sous forme de dent individuelle solitaire (24) agencée sur la mâchoire fixe (3), et sous forme de rangée de dents (25) s'étendant sur la mâchoire pivotante (4), le long de l'évidement (13).

7. Instrument de coupe médical selon l'une des revendications 1 à 6, **caractérisé en ce que** l'arête de coupe (15) orientée vers l'autre mâchoire (3) est d'une configuration en forme de U et agencée sur la mâchoire déplaçable (4) de manière telle, que l'arête de coupe (15) s'étende de l'extrémité distale de la mâchoire déplaçable (4) vers les deux zones latérales de la mâchoire déplaçable (4).

8. Instrument de coupe médical selon l'une des revendications 1 à 6, **caractérisé en ce que** dans la zone de l'arête de coupe (15) agencée sur la mâchoire déplaçable (4), un évidement (18) est formé dans l'autre mâchoire (3) en vue d'accueillir l'arête de coupe (15).

9. Instrument de coupe médical selon la revendication 8, **caractérisé en ce que** l'évidement (18) est réalisé sous forme de creux ou d'ouverture de passage (18).

10. Instrument de coupe médical selon la revendication 8 ou la revendication 9, **caractérisé en ce que** la surface de serrage (14) sur la mâchoire déplaçable (4) est agencée derrière l'arête de coupe (15), vers le côté proximal, et/ou la surface de serrage (14) sur l'autre mâchoire (3) est agencée derrière l'évidement (18), vers le côté proximal.

11. Instrument de coupe médical selon l'une des revendications 1 à 10, **caractérisé en ce que** l'extrémité distale (19) de l'autre mâchoire (3), qui dépasse de la mâchoire déplaçable (4) dans la direction axiale, est d'une configuration arrondie.

12. Instrument de coupe médical selon l'une des revendications 1 à 11, **caractérisé en ce que** dans la région de la poignée de manoeuvre (7), est agencé un mécanisme à levier (22), qui forme une butée variable pour la fermeture des mâchoires (3 et 4), en produisant ainsi une modification de la profondeur de coupe.

13. Instrument de coupe médical selon la revendication 12, **caractérisé en ce que** sur la poignée de manoeuvre (7) est agencée une graduation (23) pour déterminer la profondeur de coupe.
